# EUROPÄISCHE PATENTSCHRIFT

(11) **EP 3 683 500 B1**
(45) Veröffentlichungstag und Bekanntmachung des Hinweises auf die Patenterteilung: **06.10.2021**
(21) Anmeldenummer: 20151894.1
(22) Anmeldetag: 15.01.2020
(51) Int. Cl.: F23N 1/02, F23N 5/00, F23N 5/12, F23N 5/18

(54) **VERFAHREN ZUR REGELUNG EINES GASGEMISCHES UNTER NUTZUNG EINES GASSENSORS UND EINES GASGEMISCHSENSORS**
METHOD FOR REGULATING A GAS MIXTURE USING A GAS SENSOR AND A GAS MIXTURE SENSOR
PROCÉDÉ DE RÉGULATION D'UN MÉLANGE GAZEUX À L'AIDE D'UN CAPTEUR DE GAZ ET D'UN CAPTEUR DE MÉLANGE GAZEUX

(30) Priorität: 17.01.2019 DE 102019101191
(43) Veröffentlichungstag der Anmeldung: 22.07.2020
(73) Patentinhaber: ebm-papst Landshut GmbH, 84030 Landshut (DE)
(72) Erfinder: Henrich, Hartmut, 49076 Osnabrück (DE); Wald, Stephan, 48341 Altenberge (DE); Hermann, Jens, 49076 Osnabrück (DE)
(74) Vertreter: Staeger & Sperling Partnerschaftsgesellschaft mbB

(56) Entgegenhaltungen:
- EP-A1- 2 574 918
- WO-A1-2019/185181
- DE-U1-202018 101 271

## Beschreibung

Die Erfindung betrifft ein Verfahren zur Regelung eines Gasgemisches bei einem brenngasbetriebenen Heizgerät.

Gattungsbildende Verfahren sind aus dem Stand der Technik bekannt, beispielsweise aus der Offenbarung gemäß der Druckschrift WO2006/000366A1.

Stand der Technik ist zudem eine Verbrennungsregelung nach dem sog. SCOT-Verfahren, bei dem die Steuerung der dem Brenner des Heizgerätes zugeführte Luftmenge entsprechend der Brennerleistung erfolgt. Dabei wird eine Flammensignalmessung mittels eines ionisationssensors durchgeführt und das Gas-Luftgemisch auf einen in einer Kennlinie hinterlegten Soll-Ionisationsmesswert geregelt. Beim SCOT-Verfahren ist jedoch nachteilig, dass bei kleinen Brennerleistungen das Flammensignal stark absinkt und die Regelung damit unzuverlässig wird. Zudem ist der Adaptionsaufwand, insbesondere zur Anpassung der Brennergeometrie hoch und die Brennerleistung kann nur ungenau über die Gebläsedrehzahl eines den Luftvolumenstrom für das Gas-Luft-Gemisch liefernden Gebläses bestimmt werden.

Ferner ist im Stand der Technik eine auf die Anmelderin zurückgehende elektronische Gemischregelung durch einen thermischen Gasmassenstromsensor bekannt, mit dem die Brenngaseigenschaften erfasst werden können. Dabei misst der Gasmassenstromsensor im Brenngas den Brenngas-Volumenstrom und über ein Steuergerät wird über die thermische Leitfähigkeit aus einer Referenztabelle die Brenngasart ermittelt. Anschließend wird die erforderliche Luftmenge entsprechend dem ermittelten Luftbedarf errechnet und eingeregelt. Dabei ist jedoch aufwendig, dass alle Eingangsgrößen, d.h. der Gasvolumenstrom, der Luftvolumenstrom und die Brenngaseigenschaften gemessen und mithin überwacht werden müssen. Druckschriftlicher Stand der Technik zu dem vorliegenden technischen Gebiet ist aus den Dokumenten WO 2019/185181 A1, welches Stand der Technik unter Artikel 54(3) EPÜ darstellt, DE 20 2018 101271 U1 und EP 2 574 918 A1 bekannt.

Der Erfindung liegt die Aufgabe zugrunde, ein Verfahren zur Regelung eines Gasgemisches bei einem brenngasbetriebenen Heizgerät bereitzustellen, das Änderungen der Eigenschaften des dem Brenngas zugeführten Gases erkennt und Regelparameter gezielt anpasst.

Diese Aufgabe wird durch die Merkmalskombination gemäß Anspruch 1 gelöst.

Erfindungsgemäß wird ein Verfahren zur Regelung eines Gasgemisches gebildet aus einem Gas und einem Brenngas bei einem brenngasbetriebenen Heizgerät vorgeschlagen, bei dem das Gasgemisch erzeugt wird, indem über ein erstes Stellglied eine Gasmenge und über ein zweites Stellglied eine Brenngasmenge bereitgestellt und gemischt werden. Ein mikrothermischer Gassensor, der mindestens eine stoffliche Eigenschaft des Gases erfasst, wird mit dem Gas beaufschlagt und übermittelt ein von dem jeweiligen Gas abhängiges Sensorsignal an ein Steuergerät. Zudem wird ein mikrothermischer Gasgemischsensor, der mindestens eine stoffliche Eigenschaft des Gasgemisches erfasst, mit dem Gasgemisch beaufschlagt und übermittelt kontinuierlich ein von dem jeweiligen Gasgemisch abhängiges Sensorsignal an das Steuergerät. Bei einer Änderung des erfassten Sensorsignals des Gassensors wird das neu erfasste Sensorsignal des Gassensors mit labortechnisch gemessenen und in einer Wertetabelle im Steuergerät hinterlegten Vergleichswerten verglichen und daraus ein Sollwert des Sensorsignal des Gasgemischsensors bestimmt, ohne dass ein Mischungsverhältnis des Gasgemisches aus Brenngas und Gas verändert wird.

In der Praxis gibt es bei Heizgeräten regelmäßig den Fall, dass sich lediglich die Eigenschaft des Gases ändert, beispielsweise die relative Luftfeuchtigkeit, ohne dass sich daraus für eine weiterhin saubere Verbrennung ein neues Mischungsverhältnis für Gas und Brenngas ergeben würde. Dennoch verändert sich das Sensorsignal des Gasgemischsensors durch diese Änderung der stofflichen Eigenschaft des Gases. Zur Vermeidung einer unnötigen Anpassung des Mischungsverhältnisses wird über den Gassensor die Änderung der stofflichen Eigenschaft des Gases erfasst und lediglich der Sollwert des Sensorsignals des Gasgemischsensors angepasst. Das Mischungsverhältnis von Brenngas und Gas bleibt hierzu unverändert. Die Regelung des Gasgemisches (Verhältnis aus Brenngas und Gas) über den Modulationsbereich des Heizgeräts wird anschließend mit dem neuen Sollwert des Sensorsignals des Gasgemischsensors weitergeführt.

Diese Gasgemischregelung basiert auf einer Überwachung des Signalwerts des Gasgemischsensors. Das Steuergerät vergleicht hierzu das kontinuierlich erfasste Sensorsignal des Gasgemischsensor mit einem Sollwert des Sensorsignals des Gasgemischsensor und steuert bei einer Abweichung des erfassten Sensorsignals des Gasgemischsensor mit dem Sollwert des Sensorsignals mindestens eines der ersten und zweiten Stellglieder an, um dadurch das Gasgemisch durch Erhöhung oder Verringerung der Gasmenge und/oder Erhöhung oder Verringerung der Brenngasmenge anzupassen, bis der Sollwert des Sensorsignals des Gasgemischsensor erreicht ist.

Ein wesentlicher Punkt ist die Messung der mindestens einen stofflichen Eigenschaft des Gasgemisches sowie der mindestens einen stofflichen Eigenschaft des Gases. Eine Veränderung der Gasmenge oder der Brenngasmenge würde sofort durch eine Veränderung der stofflichen Eigenschaften am Gasgemischsensor erkannt werden. Eine Veränderung der stofflichen Eigenschaften des Gasgemisches am Gasgemischsensor kann über das Steuergerät unmittelbar ausgeregelt werden. Eine Veränderung der stofflichen Eigenschaften des Gases wird unmittelbar befasst, um den Sollwert des Sensorsignals des Gasgemischsensors anzupassen.

Die von dem mikrothermischen Gasgemischsensor erfasste stoffliche Eigenschaft des Gasgemisches ist vorzugsweise die Wärmeleitfähigkeit oder die Temperaturleitfähigkeit des Gasgemisches. Es können jedoch auch mehrere dieser stofflichen Eigenschaften erfasst werden, so dass eine genauere Zuordnung der Mehrzahl der Eigenschaften auf das Gasgemisch möglich ist. Die von dem mikrothermischen Gassensor erfasste stoffliche Eigenschaft des Gases entspricht denen des Gasgemischsensors.

Eine andere Möglichkeit besteht auf dem Funktionsprinzip der Ultraschallmessung zur Ermittlung der jeweils gasgemischabhängig vorliegenden spezifischen Schallgeschwindigkeit. Als Gassensor wird ebenfalls ein entsprechender Sensor verwendet.

Das Verfahren ist erfindungsgemäß dadurch gekennzeichnet, dass der Sollwert des Sensorsignals in Abhängigkeit einer Zusammensetzung des Gases oder des Brenngases durch das Steuergerät angepasst wird. Ändert sich die Zusammensetzung des Brenngases (z.B. von Propan auf Butan), verändern sich die gemessenen Eigenschaften des Gasgemisches. Zusätzlich benötigen andere Zusammensetzungen an Brenngas für eine optimale Verbrennung auch andere Luftmengen. Es ist somit auch ein neues Mischungsverhältnis zwischen Gas und Brenngas erforderlich.

Eine derartige Anpassung des Sollwerts des Sensorsignals erfolgt durch einen Kalibrierprozess. Hierfür werden vom Steuergerät das erste Stellglied der Gasmenge oder das zweite Stellglied der Brenngasmenge soweit verändert, bis das gewünschte Ergebnis erreicht wird. Der ursprüngliche Sollwert wird für die weitere Gemischregelung durch das neue gemessene Sensorsignal ersetzt.

Erfindungsgemäß erfolgt der Kalibrierprozess durch eine Ionisationsstromregelung eines Flammensignals eines Brenners des Heizgerätes, bis ein Ionisationssollwert erreicht ist. Hierfür wird zunächst eine stöchiometrische Verbrennung des Brenners des Heizgerätes eingestellt. Über eine lonisationssonde werden das Flammensignal des Brenners des Heizgerätes und dadurch ein entsprechender lonisationsstrom erfasst. Bei der stöchiometrischen Verbrennung ist der lonisationsstrom maximal. Aus diesem Wert des lonisationsstroms wird mit einer labortechnisch ermittelten Prozentzahl ein lonisationssollwert berechnet und als künftiger lonisationsstromsollwert abgespeichert, der bei der gewünschten Verbrennung erreicht werden muss. Anschließend wird ausschließlich die Gasmenge um einen vorbestimmten Faktor reduziert, um den Brenner mit dem gewünschten Gasgemisch bei dem vorbestimmten Ionisationssollwert zu betreiben.

Das Verfahren ist ferner dadurch gekennzeichnet, dass beim Erreichen des lonisationssollwerts die mindestens eine stoffliche Eigenschaft des Gasgemisches mittels des Gasgemischsensors gemessen und als neuer Sollwert des Sensorsignals im Steuergerät hinterlegt wird. Der neue Sollwert wird für die weitere Regelung verwendet und ersetzt den bisherigen Sollwert.

Der Kalibrierprozess erfolgt vorzugsweise bei Unplausibilitäten des Sensorsignals des Gasgemischsensors oder in zyklischen vorbestimmten Abständen. Die Feststellung von Unplausibilitäten des Sensorsignals geschieht in einer Ausführung bei einem Start des Heizgerätes, indem zunächst ausschließlich das Gas zugeführt und der Gasgemischsensor damit beaufschlagt wird. Eine Unplausibilität liegt dann vor, wenn das von dem Gasgemischsensor gemessene Sensorsignal der stofflichen Eigenschaft, z.B. die Wärmeleitfähigkeit oder die Temperaturleitfähigkeit, nicht einem Sensorsignal für das Gas entspricht. Bei Heizgeräten dient als Gas üblicherweise die Umgebungsluft, deren stoffliche Eigenschaften bekannt sind.

Verschiedene Brenngasarten bzw. Brenngasfamilien (Erdgas, Flüssiggas) beeinflussen die stofflichen Eigenschaften des Gasgemisches auf unterschiedliche Weise. Beispielsweise nimmt bei einer Zudosierung von Flüssiggas zu Luft die Wärmeleitfähigkeit ab, bei einer Zudosierung von Erdgas zu Luft nimmt die Wärmeleitfähigkeit zu. In einer Weiterbildung des Verfahrens ist deshalb vorgesehen, dass bei einem Start des Heizgerätes zunächst ausschließlich das Gas, vorzugsweise Luft, zugeführt und der Gasgemischsensor damit beaufschlagt wird. Anschließend wird das Brenngas zugeführt, das Gasgemisch erzeugt und der Gasgemischsensor mit dem Gasgemisch beaufschlagt. Aus der Änderung des Sensorsignals bei der Zuführung des Brenngases wird die Gasart des Brenngases festgestellt. Anschließend passt das Steuergerät das Gasgemisch in Abhängigkeit von der festgestellten Gasart des Brenngases an, bis der Sollwert des Sensorsignals erreicht ist. Vorteilhafterweise kann damit die Startleistung sofort nach Erkennen der Gasfamilie vom Steuergerät über die Stellung des Stellglieds des Brenngases auf einen günstigen Startpunkt gesteuert werden und das Zündgemisch zum Brennerstart wird schneller und präziser erreicht.

Das Verfahren macht sich ferner den vorstehend beschriebenen Effekt zu Zunahme bzw. Abnahme des Sensorsignals bei verschiedenen Brenngasen zunutze und sieht vor, dass aus der Änderung des Sensorsignals bei der Zuführung des Brenngases die Wirkungsrichtung der Regelung erfasst und daraus festgelegt wird, ob zur Erreichung des Sollwert des Sensorsignals die zugeführte Brenngasmenge erhöht oder erniedrigt wird. Ziel ist stets eine saubere Verbrennung mit dem dafür nötigen Gasgemisch.

Das Gas ist vorzugsweise Luft, das Brenngas vorzugsweise Flüssiggas oder Erdgas oder ein beliebiges Brenngasgesmisch.

Eine Weiterbildung des Verfahrens umfasst ferner eine Ausführung für den Fall, dass die thermischen Eigenschaften des Brenngases zu dicht an den thermischen Eigenschaften der Luft liegen und keine zuverlässige Gemischregelung möglich ist, da jede Veränderung sowohl der Luftmenge als auch der Gasmenge keine Signalveränderung am Gasgemischsensor bewirkt. Dieser Fall kann vorliegen, wenn beispielhaft Mischbrenngase zur Verbrennung eingesetzt werden, die zufällig die gleichen physikalischen stofflichen Eigenschaften wie Luft haben. Dieser Zustand wird von dem Steuergerät sowohl beim Start des Heizgerätes wie auch bei einer Kalibration durch eine Plausibilitätskontrolle dadurch erkannt, dass bei beliebiger Änderung der Luft oder der Gasmenge keine wesentliche Änderung am Sensorsignal gemessen wird. In diesem Fall kann das Steuergerät die Gemischregelung über den Gasgemischsensor vorübergehend ausschalten und mit reduziertem Modulationsbereich ausschließlich über die für die Kalibration beschriebene lonisationsstromregelung steuern. Sobald die Brenngasbeschaffenheit wieder eine Regelung über den im Gasgemisch positionierten Gasgemischsensor ermöglicht, wird die Gemischregelung über den Gasgemischsensor fortgesetzt.

In einer Ausführungsvariante ist vorgesehen, dass die von dem Gassensor erfassten stofflichen Eigenschaften des Gases kontinuierlich auf Änderungen geprüft werden und das Steuergerät den Sollwert des Sensorsignals des Gasgemischsensors kontinuierlich anpasst.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
- Fig. 1: ein prinzipieller Aufbau zur Durchführung des Verfahrens,
- Fig. 2: einen Aufbau eines Heizgerätes zur Durchführung des Verfahrens,
- Fig. 3: eine Regelungskennlinie des Sensorsignals des Gasgemischsensors,
- Fig. 4: eine Regelungskennlinie des Sensorsignals des Gasgemischsensors,
- Fig. 5: Reglungskennlinien vor und nach einer Veränderung der Gaseigenschaften,
- Fig. 6: Reglungskennlinien vor und nach einer Veränderung der Brenngaseigenschaften,
- Fig. 7: eine Kennlinie der Ionisationsstromregelung.

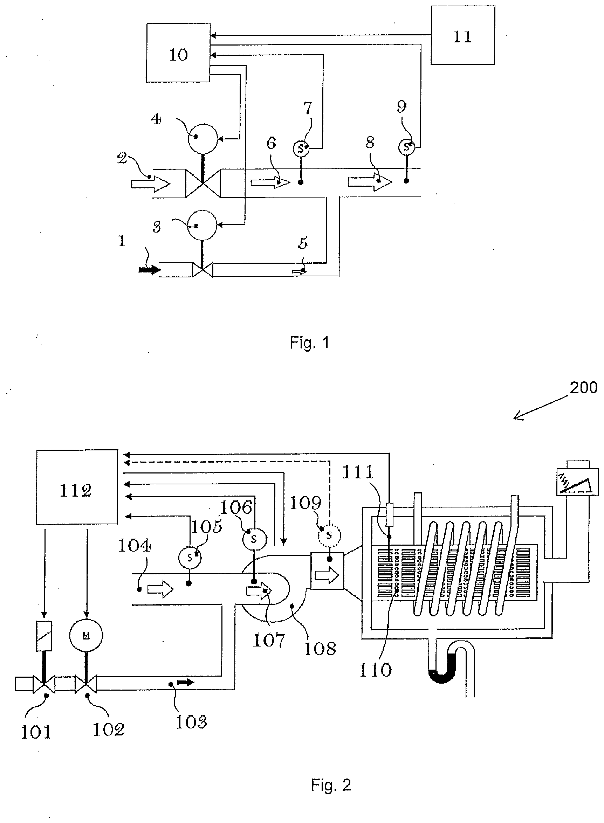

In Figur 1 ist ein prinzipieller Aufbau zur Durchführung des Verfahrens aufgezeigt. In der nachfolgenden Figurenbeschreibung wird als Gas stets Luft angenommen, auch wenn theoretisch auch andere Gase verwendet werden können.

Über das Steuergerät 10 werden das Stellglied 4 zur Zuführung einer steuerbaren Menge an Luft 2 und das Stellglied 3 zur Zuführung einer steuerbaren Menge an Brenngas 1 in ihren jeweiligen Öffnungsstellungen geregelt, um das Gasgemisch 8 in einem bestimmten Brenngas-Luftgemisch-Verhältnis zu erzeugen. Im Bereich des Gasgemisches 8 ist der Gasgemischsensor 9 positioniert und wird mit dem Gasgemisch 8 beaufschlagt. Im Bereich des geregelten Luft 6 ist der Gassensor 7 positioniert, der ausschließlich mit Luft beaufschlagt wird. Die bezüglich der Menge geregelte Luft und das bezüglich der Menge geregelte Brenngas 5 sind im Bereich des Gasgemischsensors 9 homogen gemischt. Jeder der beiden Sensoren 7, 9 ist ausgebildet, die physikalischen stofflichen Eigenschaften z.B. der Wärmeleitfähigkeit, Temperaturleitfähigkeit und/ mit anderen Sensoren der Schallgeschwindigkeit zu messen. Über eine Prozessüberwachungseinheit 11 werden das Steuergerät 10 und die Regelung überwacht. Zudem sind über Pfeile die Signalleitungen zu dem und von dem Steuergerät 10 gezeigt, welches die Regelung des Gasgemisches 8 verarbeitet.

Figur 2 zeigt eine konkrete Ausführungsform eines brenngasbetriebenen Heizgerätes 200 mit einem Gassicherheitsventil 101, einem Gasregelventil 102 als Stellglied der Menge an Brenngas 103, einem Mischgebläse 108 zur Ansaugung von Luft 104 und Mischung mit dem Brenngas 103 zur Erzeugung des Gasgemisches 107. Über die Drehzahl des Mischgebläses 108 ist die Luftmenge anpassbar; es stellt mithin das Stellglied für die Luftzufuhr. Das Heizgerät 200 umfasst den mikrothermischen Gasgemischsensor 106, wobei ein zweiter Gasgemischsensor 109 als alternative Einbauposition im Ausblasbereich des Mischgebläses 108 dargestellt ist. Grundsätzlich wird jedoch kein zweiter Gasgemischsensor benötigt. Das Heizgerät 200 umfasst ferner den mikrothermischen Gassensor 105 im Bereich der Luftzufuhr. Das Mischgebläse 108 fördert das Gasgemisch 107 zum Brenner 110, an dem die Ionisationselektrode 111 verbaut ist, um die Brennerflamme zu überwachen. Zudem sind über Pfeile die Signalleitungen zu dem und von dem Steuergerät 112 gezeigt, welches die Regelung des Gasgemisches 107 verarbeitet.

Im Folgenden wird auf die Bauteile des prinzipiellen Aufbaus gemäß Figur 1 Bezug genommen, die jedoch unmittelbar auf das Heizgerät 200 gemäß Figur 2 übertragbar sind.

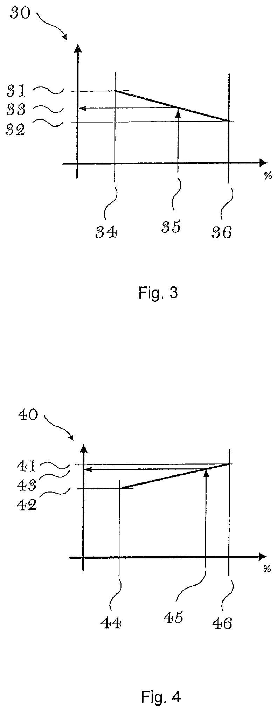

In Figur 3 ist in einem Diagramm 30 ein für die Regelung verwendeter vereinfachter linearer Zusammenhang zwischen dem von dem Gasgemischsensor 9 erfassten Sensorsignal 31 bei reiner Luft 2 (Bezugszeichen 34 entspricht 100% Luft) und dem Sensorsignal 32 bei reinem Brenngas 1 (Bezugszeichen 36 entspricht 100% Brenngas) dargestellt. Für das Gasgemisch 8 (Bezugszeichen 35 entspricht 60% Luft und 40% Brenngas) liegt das Sensorsignal 33 dazwischen. Die Mengen an Luft 2 und Brenngas 1 werden über die jeweiligen Stellglieder 3 und/oder 4 solange angepasst, bis die vom Prozess erforderlichen Gemischeigenschaften des gewünschten Mischungsverhältnisses vom Gasgemischsensor 8 detektiert werden. Figur 3 zeigt einen linearen Verlauf der Kennlinie des Sensorsignals, es sind jedoch auch nicht-lineare Kennlinien möglich, die beispielsweise über Wertetabellen eine Regelung zu den entsprechenden Positionen der Stellglieder 3, 4 ermöglichen.

Gemäß Figur 3 sinkt das Sensorsignal, je mehr Brenngas 1 zugeführt wird. Das Sensorsignal wird beispielhaft als abhängig von der Wärmeleitfähigkeit als stoffliche Eigenschaft des Gasgemisches 8 dargestellt, wobei das Brenngas beispielsweise Flüssiggas ist und die Wärmeleitfähigkeit von Flüssiggas niedriger ist als diejenige von Luft. Es gibt jedoch auch Gasarten, bei denen die Wirkrichtung der Regelung umgekehrt ist, wie in Figur 4 gezeigt. Hier ist das Brenngas 1 Erdgas, dessen Wärmeleitfähigkeit höher ist als diejenige von Luft. Im Diagramm 40 gemäß Figur 4 ein für die Regelung verwendeter vereinfachter linearer Zusammenhang zwischen dem von dem Gasgemischsensor 9 erfassten Sensorsignal 41 bei reiner Luft 2 (Bezugszeichen 44 entspricht 100% Luft) und dem Sensorsignal 42 bei reinem Brenngasas 1 (Bezugszeichen 46 entspricht 100% Brenngas/Erdgas) dargestellt. Für das Gasgemisch 8 (Bezugszeichen 45 entspricht 75% Luft und 25% Brenngas/Erdgas) liegt das Sensorsignal 43 dazwischen, jedoch nahe dem Sensorsignal 41 reinen Brenngases 1. Für eine Regelung mit Erdgas wird vom Steuergerät 10 aus der Signaländerung des Gasgemischsensors 9 bei der Erhöhung der Brenngasmenge die Wirkungsrichtung der Regelung bestimmt und für die weitere Gemischregelung zu Grunde gelegt.

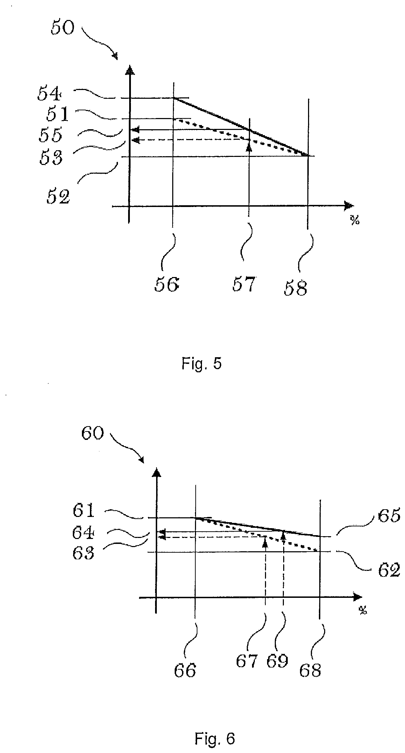

Die Ermittlung des Sollwerts des Sensorsignals des Gasgemischsensors 10 ist im Diagramm 50 gemäß Figur 5 schematisch dargestellt. Aus der durch den Gassensor 7 gemessenen stofflichen Eigenschaft, z.B. bei Veränderung der relativen Luftfeuchtigkeit, wird ein neuer Sensorsignal-Sollwert ermittelt. Das Mischungsverhältnis 57 aus Brenngas 2 und Luft 1 bleibt jedoch unverändert. Wenn beispielhaft bei reiner Luft 1 (Bezugszeichen 56 entspricht 100% Luft; Bezugszeichen 58 reinem Brenngas) sich aufgrund einer Veränderung der Beschaffenheit das Sensorsignal, z.B. bei einer Änderung der relativen Luftfeuchtigkeit, von dem ursprünglichen Wert (Bezugszeichen 51) auf einen neuen Wert (Bezugszeichen 54) ändert, wird vom Steuergerät 11 der Gemischsensor-Sollwert von dem alten Wert (Bezugszeichen 53) auf den neuen Wert (Bezugszeichen 55) unter Nutzung des Signalwerts des Gassensors 7 hochgerechnet oder mit Werten aus einer im Steuergerät 11 hinterlegten Wertetabellen korrigiert. Die Bezugszeichen 52 und 54 bezeichnen die Sensorsignale bei reiner Luft bzw. bei reinem Brenngas.

Figur 6 zeigt ein Diagramm 60 zur Darstellung der Kalibration, wenn sich beispielsweise die Beschaffenheit des Brenngases 1 so ändert, dass eine neue Gasgemischzusammensetzung erforderlich ist, um eine optimale Verbrennung zu gewährleisten. In Figur 6 verändert sich das Brenngas als Beispiel von Propan auf Butan. Die Bezugszeichen 66 und 68 bestimmen den Bereich zwischen 100% Luft und 100% Brenngas, wobei der Signalwert 61 bei 100% Luft vorliegt. Bei der Kalibrierung wird das Gasgemisch 8 von dem ursprünglichen Mischungsverhältnis 67 mit dem zugehörigen Signalwert 63 auf das neue Mischungsverhältnis 69 mit dem zugehörigen Signalwert 64 verändert. Durch die Erfassung der stofflichen Eigenschaften sowohl der Luft 2 als auch des Brenngases 1 über jeweils einen eigenen Sensor kann die Wirkungsrichtung der Regelung vorbestimmt werden und das Gasgemisch 8 für die nachfolgende Regelung der Brenngasmenge und/oder Luftmenge voreingestellt werden. In den Figuren 5 und 6 ist die Situation der ursprünglichen Beschaffenheit des Brenngases gestrichelt, die neue Situation mit durchgezogenen Linien und Pfeilen dargestellt.

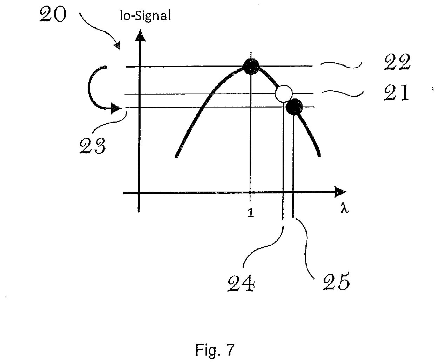

Figur 7 zeigt ein Diagramm 20 zur Kalibrierung mittels lonisationsstromregelung mit einer Kennlinie des von der lonisationselektrode in der Brennerflamme erfassten lonisationssignals (lo-Signal) gegenüber dem Brenngas-Luftverhältnis A. Da der prinzipielle Aufbau gemäß Figur 1 keine lonisationselektrode zeigt, wird nachfolgend auf das Heizgerät 200 gemäß Figur 2 verwiesen. Vom Steuergerät 112 wird während des Brennerbetriebes die Menge an Luft 105 auf einen vorgegebenen Wert gesteuert, das lonisationssignal an der Ionisationselektrode 111 des Brenners 110 gemessen und die Menge an Brenngas 103 soweit erhöht, bis das lonisationssignal von dem ursprünglich vorhandenen Ionisationswert 21 bei einem Brenngas-Luftverhältnis 24 auf das Maximum 22 angestiegen ist. Aus diesem Wert wird mit einer labortechnisch ermittelten Prozentzahl der lonisationssollwert 23 berechnet und als künftiger lonisationsstrom-Sollwert abgespeichert, der das gewünschte Brenngas-Luftverhältnis 25 mit höherem Luftüberschuss erreicht werden muss.

## Patentansprüche

1. Verfahren zur Regelung eines Gasgemisches (8, 107) gebildet aus einem Gas (2, 104) und einem Brenngas (1, 103) bei einem brenngasbetriebenen Heizgerät, wobei das Gasgemisch erzeugt wird, indem
über ein erstes Stellglied (4, 108) eine Gasmenge und
über ein zweites Stellglied (3, 102) eine Brenngasmenge bereitgestellt und gemischt werden, wobei
ein mikrothermischer Gassensor (7, 105), der mindestens eine stoffliche Eigenschaft des Gases (2, 104) erfasst, mit dem Gas beaufschlagt wird und ein von dem jeweiligen Gas abhängiges Sensorsignal an ein Steuergerät (10, 112) übermittelt,
wobei ein mikrothermischer Gasgemischsensor (9, 106, 109), der mindestens eine stoffliche Eigenschaft des Gasgemisches (8, 107) erfasst, mit dem Gasgemisch beaufschlagt wird und kontinuierlich ein von dem jeweiligen Gasgemisch abhängiges Sensorsignal an das Steuergerät (10, 112) übermittelt, wobei bei einer Änderung des erfassten Sensorsignals des Gassensors das neu erfasste Sensorsignal des Gassensors mit labortechnisch gemessenen und in einer Wertetabelle im Steuergerät (10, 112) hinterlegten Vergleichswerten verglichen und daraus ein Sollwert des Sensorsignal des Gasgemischsensors (9, 106, 109) bestimmt wird, ohne dass ein Mischungsverhältnis des Gasgemisches (9, 108) aus Brenngas und Gas verändert wird,
wobei der Sollwert des Sensorsignals des Gasgemischsensors (9, 106, 109) in Abhängigkeit einer Zusammensetzung des Gases oder des Brenngases durch das Steuergerät (10, 112) angepasst wird und die Anpassung des Sollwerts des Sensorsignals des Gasgemischsensors (9, 106, 109) durch einen Kalibrierprozess erfolgt, und wobei
der Kalibrierprozess insbesondere durch eine lonisationsstromregelung eines Flammensignals eines Brenners (109) des Heizgerätes (200) erfolgt, bis ein Ionisationssollwert erreicht ist.

2. Verfahren nach Anspruch 1, wobei das Steuergerät (10 ,112) das kontinuierlich erfasste Sensorsignal des Gasgemischsensor (9, 106, 109) mit einem Sollwert des Sensorsignals des Gasgemischsensor (9, 106, 109) vergleicht und bei einer Abweichung des erfassten Sensorsignals des Gasgemischsensor (9, 106, 109) mit dem Sollwert des Sensorsignals mindestens eines der ersten und zweiten Stellglieder ansteuert und dadurch das Gasgemisch (8, 105) durch Erhöhung oder Verringerung der Gasmenge und/oder Erhöhung oder Verringerung der Brenngasmenge anpasst, bis der Sollwert des Sensorsignals des Gasgemischsensor (9, 106, 109) erreicht ist.

3. Verfahren nach Anspruch 1 oder 2, wobei die von dem mikrothermischen Gasgemischsensor (9, 106, 109) erfasste stoffliche Eigenschaft des Gasgemisches die Wärmeleitfähigkeit und/oder die Temperaturleitfähigkeit des Gasgemisches ist/sind.

4. Verfahren nach einem der vorigen Ansprüche 1 - 3, wobei
die von dem mikrothermischen Gassensor (7, 105) erfasste stoffliche Eigenschaft des Gases die Wärmeleitfähigkeit und/oder die Temperaturleitfähigkeit des Gases ist/sind.

5. Verfahren nach einem der Ansprüche 1 - 4, wobei
beim Erreichen des lonisationssollwerts die mindestens eine stoffliche Eigenschaft des Gasgemisches (8, 105) mittels des Gasgemischsensors (9, 106, 109) gemessen und als neuer Sollwert des Sensorsignals im Steuergerät (10 ,112) hinterlegt wird.

6. Verfahren nach einem der Ansprüche 1 - 5, wobei
der Kalibrierprozess bei Unplausibilitäten des Sensorsignals des Gasgemischsensors (9, 106, 109) oder zyklisch erfolgt.

7. Verfahren nach dem vorigen Anspruch, wobei eine Feststellung der Unplausibilitäten des Sensorsignals bei einem Start des Heizgerätes erfolgt, indem zunächst ausschließlich das Gas (2, 104) zugeführt und der Gasgemischsensor (9, 106, 109) damit beaufschlagt wird, wobei eine Unplausibilität vorliegt, wenn das von dem Gasgemischsensor (9, 106, 109) gemessene Sensorsignal nicht einem Sensorsignal für das bekannte Gas (2, 104) entspricht.

8. Verfahren nach einem der vorigen Ansprüche, wobei bei einem Start des Heizgerätes zunächst ausschließlich das Gas (2, 104) zugeführt und der Gasgemischsensor (9, 106, 109) damit beaufschlagt wird, wobei anschließend das Brenngas (1, 103) zugeführt, das Gasgemisch (8, 105) erzeugt und der Gasgemischsensor (9, 106, 109) mit dem Gasgemisch (8, 105) beaufschlagt wird, wobei aus einer Änderung des Sensorsignals bei der Zuführung des Brenngases die Gasart des Brenngases (1, 103) festgestellt und das Steuergerät (10, 112) das Gasgemisch (8, 105) in Abhängigkeit von der festgestellten Gasart des Brenngases (1, 103) anpasst, bis der Sollwert des Sensorsignals erreicht ist.

9. Verfahren nach dem vorigen Anspruch, wobei aus der Änderung des Sensorsignals bei der Zuführung des Brenngases (1, 103) die Wirkungsrichtung der Regelung erfasst und daraus festgelegt wird, ob zur Erreichung des Sollwerts des Sensorsignals die zugeführte Brenngasmenge erhöht oder erniedrigt wird.

10. Verfahren nach Anspruch 8 oder 9, wobei die von dem Gassensor erfassten stofflichen Eigenschaften des Gases kontinuierlich auf Änderungen geprüft werden und das Steuergerät (10 ,112) den Sollwert des Sensorsignals des Gasgemischsensors (9, 106, 109) kontinuierlich anpasst.

11. Verfahren nach einem der vorigen Ansprüche, wobei das Gas (2, 104) Luft und das Brenngas (1, 103) beliebig wählbar ist.

## Claims

1. Method for regulating a gas mixture (8, 107) formed from a gas (2, 104) and a fuel gas (1, 103) in a fuel gas-operated heating appliance, wherein the gas mixture is created by providing and mixing a gas quantity by way of a first control element (4, 108) and a fuel gas quantity by way of a second control element (3, 102), wherein a microthermal gas sensor (7, 105), which detects at least one material property of the gas (2, 104), is exposed to the gas and relays a sensor signal dependent on the respective gas to a controller (10, 112), wherein a microthermal gas mixture sensor (9, 106, 109), which detects at least one material property of the gas mixture (8, 107), is exposed to the gas mixture and continuously relays a sensor signal dependent on the respective gas mixture to the controller (10, 112), wherein, upon change in the detected sensor signal of the gas sensor, a newly detected sensor signal of the gas sensor is compared with reference values which have been measured in the laboratory and saved in a table of values in the controller (10, 112) and from this a target value of the sensor signal of the gas mixture sensor (9, 106, 109) is determined without a mixture ratio of the gas mixture (9, 108) composed of fuel gas and gas being changed, wherein the target value of the sensor signal of the gas mixture sensor (9, 106, 109) is adapted in dependence on a composition of the gas or the fuel gas by the controller (10, 112) and the adapting of the target value of the sensor signal of the gas mixture sensor (9, 106, 109) is done by a calibration process and wherein the calibration process is done in particular by an ionization current regulation of a flame signal of a burner (109) of the heating appliance (200), until an ionization target value is reached.

2. Method according to claim 1, wherein the controller (10, 112) compares the continuously detected sensor signal of the gas mixture sensor (9, 106, 109) with a target value of the sensor signal of the gas mixture sensor (9, 106, 109) and upon deviation of the detected sensor signal of the gas mixture sensor (9, 106, 109) from the target value of the sensor signal it actuates at least one of the first and second control elements and thereby adapts the gas mixture (8, 105) by increasing or decreasing the gas quantity and/or raising or lowering the fuel gas quantity until the target value of the sensor signal of the gas mixture sensor (9, 106, 109) is reached.

3. Method according to claim 1 or 2, wherein the material property of the gas mixture detected by the microthermal gas mixture sensor (9, 106, 109) is/are the thermal conductivity and/or the temperature diffusivity of the gas mixture.

4. Method according one of the preceding claims 1 to 3, wherein the material property of the gas detected by the microthermal gas sensor (7, 105) is/are the thermal conductivity and/or the temperature diffusivity of the gas.

5. Method according to one of the claims 1 to 4, wherein upon reaching the ionization target value, the at least one material property of the gas mixture (8, 105) is measured by means of the gas mixture sensor (9, 106, 109) and saved as a new target value of the sensor signal in the controller (10,112).

6. Method according to one of the claims 1 to 5, wherein the calibration process is done in the event of implausibilities of the sensor signal of the gas mixture sensor (9, 106, 109) or in a cyclical manner.

7. Method according to the preceding claim, wherein a determination of the implausibilities of the sensor signal is done at the starting of the heating appliance by first of all supplying only the gas (2, 104) and exposing the gas mixture sensor (9, 106, 109) to it, wherein an implausibility is present if the sensor signal as measured by the gas mixture sensor (9, 106, 109) does not match a sensor signal for the known gas (2, 104).

8. Method according to one of the preceding claims, wherein upon starting the heating appliance at first only the gas (2, 104) is supplied and the gas mixture sensor (9, 106, 109) is exposed to it, wherein then the fuel gas (1, 103) is supplied, the gas mixture (8, 105) is created, and the gas mixture sensor (9, 106, 109) is exposed to the gas mixture (8, 105), wherein the nature of the fuel gas (1, 103) is determined from a change in the sensor signal when the fuel gas is supplied, and the controller (10, 112) adapts the gas mixture (8, 105) in dependence on the ascertained nature of the fuel gas (1, 103) until the target value of the sensor signal is reached.

9. Method according to the preceding claim, wherein the direction of action of the regulation is detected from the change in the sensor signal when the fuel gas (1, 103) is supplied and from this it is determined whether the supplied fuel gas quantity is increased or decreased in order to reach the target value of the sensor signal.

10. Method according to claim 8 or 9, wherein the material properties of the gas as detected by the gas sensor are checked continuously for changes and the controller (10, 112) continuously adapts the target value of the sensor signal of the gas mixture sensor (9, 106, 109).

11. Method according to one of the preceding claims, wherein the gas (2, 104) is air and the fuel gas (1, 103) can be chosen arbitrarily.

## Revendications

1. Procédé de régulation d'un mélange gazeux (8, 107) composé d'un gaz (2, 104) et un gaz combustible (1, 103) dans un appareil de chauffage actionné par gaz combustible, le mélange gazeux étant produit en ce que
un premier actionneur (4, 108) fournit une quantité de gaz, et
un deuxième actionneur (3, 102) fournit et mélange une deuxième quantité de gaz combustible, dans lequel
un capteur de gaz microthermique (7, 105), qui détecte au moins une propriété matérielle du gaz (2, 104), est sollicité par le gaz et transmet à un appareil de commande (10, 112) un signal de capteur en fonction du gaz respectif,
un capteur de mélange gazeux (9, 106, 109) microthermique, qui détecte au moins une propriété matérielle du mélange gazeux (8, 107), étant sollicité par le mélange gazeux et transmettant en continu à l'appareil de commande (10, 112) un signal de capteur en fonction du mélange gazeux respectif,
dans lequel, en cas de changement du signal de capteur détecté du capteur de gaz, le signal de capteur du capteur de gaz nouvellement détecté est comparé avec des valeurs de comparaison mesurées par des techniques de laboratoire et enregistrées dans une table de valeurs sur l'appareil de commande (10, 112), et une valeur de consigne du signal de capteur du capteur de mélange gazeux (9, 106, 109) est déterminée sur cette base sans pour autant modifier un rapport de mélange du mélange gazeux (9, 108) composé de gaz combustible et de gaz,
la valeur de consigne du signal de capteur du capteur de mélange gazeux (9, 106, 109) étant adaptée par l'appareil de commande (10, 112) en fonction d'une composition du gaz ou du gaz combustible, et l'adaptation de la valeur de consigne du signal de capteur du capteur de mélange gazeux (9, 106, 109) est effectuée par un processus d'étalonnage, et dans lequel
le processus d'étalonnage est effectué en particulier par une régulation de courant d'ionisation d'un signal de flamme d'un brûleur (109) de l'appareil de chauffage (200) jusqu'à ce qu'une valeur de consigne d'ionisation soit atteinte.

2. Procédé selon la revendication 1, dans lequel l'appareil de commande (10, 112) compare le signal de capteur, détecté en continu, du capteur de mélange gazeux (9, 106, 109) avec une valeur de consigne du signal de capteur du capteur de mélange gazeux (9, 106, 109), et en cas d'écart du signal de capteur détecté du capteur de mélange gazeux (9, 106, 109), pilote avec la valeur de consigne du signal de capteur au moins l'un du premier et du deuxième actionneur, et adapte ainsi le mélange gazeux (8, 105) en augmentant ou en diminuant la quantité de gaz et/ou en augmentant ou en diminuant la quantité de gaz combustible jusqu'à ce que la valeur de consigne du signal de capteur du capteur de mélange gazeux (9, 106, 109) soit atteinte.

3. Procédé selon la revendication 1 ou 2, dans lequel la propriété matérielle du mélange gazeux, détectée par le capteur de mélange gazeux (9, 106, 109) microthermique, est la conductibilité thermique et/ou la diffusion thermique du mélange gazeux.

4. Procédé selon l'une quelconque des revendications précédentes 1 à 3, dans lequel la propriété matérielle du gaz, détectée par le capteur de gaz (7, 105) microthermique, est la conductibilité thermique et/ou la diffusion thermique du gaz.

5. Procédé selon l'une quelconque des revendications 1 à 4, dans lequel lorsque la valeur de consigne d'ionisation est atteinte, ladite au moins une propriété matérielle du mélange gazeux (8, 105) est mesurée au moyen du capteur de mélange gazeux (9, 106, 109) et est enregistrée sur l'appareil de commande (10, 112) comme la nouvelle valeur de consigne du signal de capteur.

6. Procédé selon l'une quelconque des revendications 1 à 5, dans lequel le processus d'étalonnage est effectué en cas d'invraisemblances du signal de capteur du capteur de mélange gazeux (9, 106, 109) ou de manière cyclique.

7. Procédé selon la revendication précédente, dans lequel un constat des invraisemblances du signal de capteur est effectué au démarrage de l'appareil de chauffage en ce que d'abord exclusivement le gaz (2, 104) est amené et le capteur de mélange gazeux (9, 106, 109) est sollicité par celui-ci, une invraisemblance étant donnée si le signal de capteur mesuré par le capteur de mélange gazeux (9, 106, 109) ne correspond pas à un signal de capteur pour le gaz connu (2, 104).

8. Procédé selon l'une quelconque des revendications précédentes, dans lequel, au démarrage de l'appareil de chauffage, d'abord exclusivement le gaz (2, 104) est amené et le capteur de mélange gazeux (9, 106, 109) est sollicité par celui-ci, ensuite le gaz combustible (1, 103) étant amené, le mélange gazeux (8, 105) étant produit et le capteur de mélange gazeux (9, 106, 109) étant sollicité par le mélange gazeux (8, 105), dans lequel, d'après un changement du signal de capteur lorsque le gaz combustible est amené, le type de gaz du gaz combustible (1, 103) est constaté, et l'appareil de commande (10, 112) adapte le mélange gazeux (8, 105) en fonction du type de gaz constaté du gaz combustible (1, 103) jusqu'à ce que la valeur de consigne du signal de capteur soit atteinte.

9. Procédé selon la revendication précédente, dans lequel, d'après le changement du signal de capteur lorsque le gaz combustible (1, 103) est amené, le sens d'action de la régulation est détecté, et on définit en conséquence si pour atteindre la valeur de consigne du signal de capteur il faut augmenter ou diminuer la quantité de gaz combustible amenée.

10. Procédé selon la revendication 8 ou 9, dans lequel les propriétés matérielles du gaz, détectées par le capteur de gaz, sont en continu vérifiées quant à des changements, et l'appareil de commande (10, 112) adapte en continu la valeur de consigne du signal de capteur du capteur de mélange gazeux (9, 106, 109).

11. Procédé selon l'une quelconque des revendications précédentes, dans lequel le gaz (2, 104) est de l'air et le gaz combustible (1, 103) peut être choisi librement.
